# EUROPEAN PATENT APPLICATION

(11) **EP 3 828 137 A1**
(43) Date of publication of application: **02.06.2021**
(21) Application number: 19841241.3
(22) Date of filing: 23.07.2019
(51) Int. Cl.: C01F 17/00, C01G 45/02

(54) **RADIATION-PROTECTIVE NANOPARTICLES**

(30) Priority: 26.07.2018 KR 20180087355
(71) Applicant: Seoul National University R & DB Foundation, Seoul 08826 (KR); Institute for Basic Science, Yuseong-gu Daejeon 34126 (KR)
(72) Inventor: HYEON, Taeghwan, Seoul 08826 (KR); PARK, Kyungpyo, Seoul 08826 (KR); LEE, Sangwoo, Seoul 08826 (KR); CHO, Mingee, Seoul 08826 (KR); HAN, Sangihn, Seoul 08826 (KR)
(74) Representative: Ter Meer Steinmeister & Partner
(86) International application number: PCT/KR2019/009065
(87) International publication number: WO 2020/022736

(57) **Abstract**

A nanoparticle for radiation protection is provided. The nanoparticle for radiation protection comprises a first metal oxide nanoparticle. The nanoparticle for radiation protection may further comprise a second metal oxide layer formed on a surface of the first metal oxide nanoparticle. The nanoparticle for radiation protection may have a polar interface between the first metal oxide nanoparticle and the second metal oxide layer.

## Description

### Technical Field

The present invention relates to a nanoparticle for radiation protection.

### Background Art

Exposure to high doses of ionizing radiation (IR) can occur in various medical fields and industries. In medical fields at least 50% of cancer patients get radiotherapy due to its cost-effectiveness and non-invasiveness. However during the radiotherapy normal tissues can also be damaged especially when they are close to the tumor mass. One example is radiation-induced hypo-salivation or xerostomia, caused when salivary glands are irradiated during the head and neck cancer (HNC) radiotherapy.

HNC is the eighth most common cancer with over 550,000 new cases reported annually and 52% of HNC patients get radiotherapy. Radiation-induced xerostomia is the biggest acute and long-term side effect in HNC radiotherapy since it is associated with key oral functions including chewing, swallowing and oral hygiene which may largely affect patients' prognosis and long-term quality of life. In addition to the medical fields utilization of nuclear power in industrial fields has been rapidly growing as the energy demand increases.

Unlike the exposure to IR during medical procedure accidental total body irradiation (TBI) cause acute life-threatening consequences such as hematopoietic syndrome and gastrointestinal syndrome. Microscopically when tissues are exposed to IR ROS is rapidly generated within a millisecond via radiolysis of water. These ROS reacts with nucleic acids and cellular components leading to the disruption of biological functions and even cellular apoptosis. Since the cells with high mitotic rate are more vulnerable to IR-induced ROS progenitor/stem cells or tissues associated with regeneration are usually compromised first and thus making the radiation-induced damage irreversible and non-regenerative.

### Disclosure

### Technical Problem

In order to solve the above mentioned problems, the present invention provides a nanoparticle for radiation protection having excellent radiation protection ability.

The present invention provides a nanoparticle for radiation protection having excellent biocompatibility.

The other objects of the present invention will be clearly understood by reference to the following detailed description and the accompanying drawings.

### Technical Solution

A nanoparticle for radiation protection according to the embodiments of the present invention comprises a first metal oxide nanoparticle.

The nanoparticle for radiation protection may comprise a second metal oxide layer formed on a surface of the first metal oxide nanoparticle.

The nanoparticle for radiation protection may have a polar interface between the first metal oxide nanoparticle and the second metal oxide layer. The polar interface may comprise (a metal ion of the first metal oxide)-O-(a metal ion of the second metal oxide) pairing.

The second metal oxide layer may be formed on the surface of the first metal oxide nanoparticle by epitaxial growth.

Oxygen vacancies of the surface of the first metal oxide nanoparticle may be increased by the second metal oxide layer.

The second metal oxide layer may be strained to expose {332} face.

The first metal oxide may comprise ceria and the second metal oxide may comprise manganese oxide.

The first metal oxide nanoparticle may have oxygen vacancies. The first metal oxide nanoparticle may have a fluorite-structured nanocrystal.

### Advantageous Effects

A nanoparticle for radiation protection according to the embodiments of the present invention can have excellent radiation protection ability and biocompatibility. The nanoparticle for radiation protection can increase the ability of scavenging reactive oxygen species and reduce systemic toxicity. In addition, the nanoparticle for radiation protection can have long-lasting stable activity and low active dose. The nanoparticle for radiation protection can be used locally and systemically to protect various radiation-induced damages. The nanoparticle for radiation protection is non-toxic or has low toxicity, and it can also directly protect tissues and cells from radiation with small dose, thereby promoting faster regeneration and recovery of functions.

### Description of Drawings

FIG. 1 shows a nanoparticle for radiation protection according to an embodiment of the present invention.
FIG. 2 shows a nanoparticle for radiation protection according to another embodiment of the present invention.
FIGS. 3 to 10 are views for explaining the characteristics of the nanoparticle for radiation protection of FIGS. 1 and 2.
FIGS. 11 to 17 are views for explaining the enzyme mimetic activity of cerium-manganese oxide nanoparticle.
FIGS. 18 to 24 are views for explaining the protection of cerium-manganese oxide nanoparticle for eSMG from radiation induced damages.
FIGS. 25 to 35 are views for explaining the protection of cerium-manganese oxide nanoparticle against IR locally irradiated in vivo.
FIGS. 36 to 49 are views for explaining the protection of cerium-manganese oxide nanoparticle against IR entirely irradiated in vivo.

### Best Mode

Hereinafter, a detailed description will be given of the present invention with reference to the following embodiments. The purposes, features, and advantages of the present invention will be easily understood through the following embodiments. The present invention is not limited to such embodiments, but may be modified in other forms. The embodiments to be described below are nothing but the ones provided to bring the disclosure of the present invention to perfection and assist those skilled in the art to completely understand the present invention. Therefore, the following embodiments are not to be construed as limiting the present invention.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, a first element, component, region, layer or section discussed below could be termed a second element, component, region, layer or section without departing from the teachings of the present invention.

The size of the element or the relative sizes between elements in the drawings may be shown to be exaggerated for more clear understanding of the present invention. In addition, the shape of the elements shown in the drawings may be somewhat changed by variation of the manufacturing process or the like. Accordingly, the embodiments disclosed herein are not to be limited to the shapes shown in the drawings unless otherwise stated, and it is to be understood to comprise a certain amount of variation.

The terms +IR and IR+, -IR and IR-, +CM and CM+, -CM and CM-used herein have following meaning. +IR and IR+ mean that radiation is applied, and -IR and IR- mean that no radiation is applied. +CM and CM+ mean that treatment by cerium-manganese oxide nanoparticles is conducted (For example, to be administered or injected into a living body), and -CM and CM- mean that no treatment by cerium-manganese oxide nanoparticles is conducted. For example, +IR/+CM and IR+CM+ represents a group that radiation is applied to and is treated with cerium-manganese oxide nanoparticles, and +IR/-CM and IR+CM- represents a group that radiation is applied to but is not treated with cerium-manganese oxide nanoparticles.

A nanoparticle for radiation protection according to one embodiment of the present invention may comprise a first metal oxide nanoparticle. The first metal oxide nanoparticle may have oxygen vacancies. The first metal oxide nanoparticle may have a fluorite-structured nanocrystal. The first metal oxide may comprise, for example, ceria (cerium oxide).

A nanoparticle for radiation protection according to another embodiment of the present invention may comprise a first metal oxide nanoparticle and a second metal oxide layer formed on a surface of the first metal oxide nanoparticle.

The nanoparticle for radiation protection may have a polar interface between the first metal oxide nanoparticle and the second metal oxide layer. The polar interface may comprise (a metal ion of the first metal oxide)-O-(a metal ion of the second metal oxide) pairing. For example, the first metal oxide may comprise ceria, the second metal oxide may comprise manganese oxide, and the polar interface may comprise Ce⁴⁺-O-Mn²⁺ pairing. That is, the nanoparticle for radiation protection may comprise ceria nanoparticle (CeO₂), manganese oxide (Mn₃O₄) layer formed on the surface of the ceria nanoparticle and Ce⁴⁺-O-Mn²⁺ pairing at the heterointerface between the ceria nanoparticle and the manganese oxide layer.

The first metal oxide nanoparticle may have oxygen vacancies. The first metal oxide nanoparticle may have a fluorite-structured nanocrystal. The second metal oxide layer may be formed on the surface of the first metal oxide nanoparticle by epitaxial growth. The oxygen vacancies of the surface of the first metal oxide nanoparticle may be increased by the second metal oxide layer. The second metal oxide layer may be strained to expose {332} face.

The nanoparticle for radiation protection may have an F_{2g} peak and an A_{1g} peak in visible Raman spectra. The surface oxygen reduction peak of the nanoparticle for radiation protection may be shown at a temperature lower than the temperature of the surface oxygen reduction peak of the first metal oxide and the second metal oxide in H₂ TPR (temperature-programmed reduction).

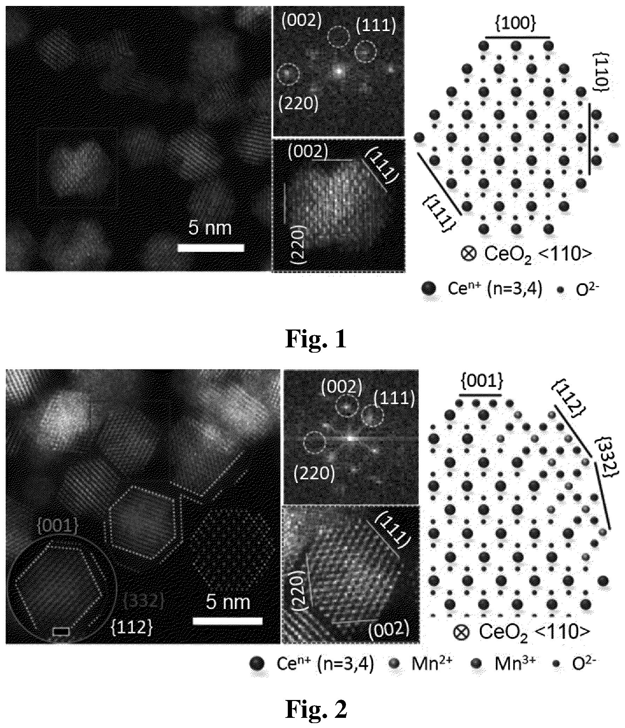

FIG. 1 shows a nanoparticle for radiation protection according to an embodiment of the present invention.

Referring to FIG. 1, the nanoparticle for radiation protection comprises ceria nanoparticle. The ceria nanoparticle may have oxygen vacancies. The ceria nanoparticle may have fluorite-structured nanocrystals, the lattice spacing of (111) plane is 3.14 Å.

The ceria nanoparticle can be formed in the following method.

A mixed solution of 0.43 g of cerium (III) nitrate, 2.7 g of oleylamine and 0.03 g of oleic acid in 15 mL of 1-dodecanol is heated in air at 120 °C. The mixed solution is aged for several minutes until it turns yellow. During the heating process, 0.3 mL of distilled water at about 90 °C is added. After the reaction, an excess of acetone, ethanol and acetonitrile are added to purify ceria nanoparticles. The ceria nanoparticles are separated by centrifugation to be collected. In this way, it is possible to form 4 nm-sized truncated octahedral ceria nanoparticles dominantly enclosed by {100} and {111} planes in 1-dodecanol solution using microemulsion method.

FIG. 2 shows a nanoparticle for radiation protection according to another embodiment of the present invention.

Referring to FIG. 2, the nanoparticle for radiation protection may comprise cerium-manganese oxide nanoparticle. The cerium-manganese oxide nanoparticle may comprise cerium oxide (ceria) and manganese oxide. The cerium-manganese oxide nanoparticle may comprise ceria nanopartice and manganese oxide layer formed on a surface of the ceria nanoparticle.

The cerium-manganese oxide nanoparticle may have a polar interface between the ceria nanopartice and the manganese oxide layer. The polar interface may comprise Ce⁴⁺-O-Mn²⁺ pairing. That is, the cerium-manganese oxide nanoparticle may comprise ceria nanoparticle (CeO₂), manganese oxide (Mn₃O₄) layer formed on the surface of the ceria nanoparticle and Ce⁴⁺-O-Mn²⁺ pairing at the heterointerface between the ceria nanoparticle and the manganese oxide layer.

The ceria nanoparticle may have oxygen vacancies. The ceria nanoparticle may have a fluorite-structured nanocrystal. The manganese oxide layer may be formed on the surface of the ceria nanoparticle by epitaxial growth. The oxygen vacancies of the surface of the ceria nanoparticle may be increased by the manganese oxide layer. The manganese oxide layer may be strained to expose {332} face. The cerium-manganese oxide nanoparticle may have high index Mn₃O₄ {332} facets induced by strain. The HAADF-STEM (High-angle annular dark-field scanning transmission electron microscopy) image in FIG. 2 shows that the morphology of the ceria nanoparticle is well preserved after the heterogeneous deposition of the manganese oxide.

The cerium-manganese oxide nanoparticle can be formed in the following method.

A mixed solution of 0.09 g of ceria nanoparticles, 1.34 g of oleylamine, 0.14 g of oleic acid, 0.26 mL of hydrochloric acid, and 15 mL of xylene is heated at 90 °C. 0.8 mL of an aqueous solution of 0.38 M manganese (II) chloride is rapidly injected into the heated mixed solution. After aging the mixed solution for 2 hours, the cerium-manganese oxide nanoparticles are washed with hexane and ethanol, and separated by centrifugation to be collected. In this way, the ceria nanoparticles can be reacted with Mn²⁺ ions to form heterostructured cerium-manganese oxide nanoparticles. The heterostructured cerium-manganese oxide nanoparticles are well dispersed in chloroform.

The crystal phase of ceria and heterostructured cerium-manganese oxide nanoparticles can be characterized by X-ray diffraction (XRD). The XRD pattern of the cerium-manganese oxide nanoparticles is nearly the same with that of the ceria nanoparticles, which means that the manganese oxide is epitaxially grown on the ceria nanoparticles without homogeneous precipitation.

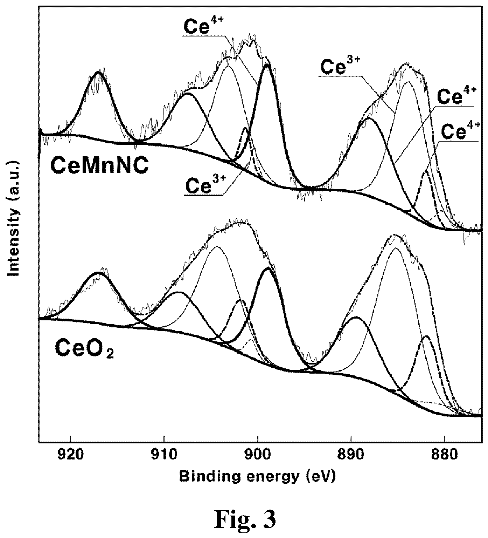

FIGS. 3 to 10 are views for explaining the characteristics of the nanoparticle for radiation protection of FIGS. 1 and 2.

The layer of manganese oxide (Mn₃O₄) has a thickness of about 1nm, which is 3 atomic layers of {112} plane when a molar ratio of Mn to Ce is between 15% and 30%. The growth mechanism of manganese oxide on ceria is studied with X-ray photoelectron spectroscopy (XPS), X-ray absorption spectra (XAS) and STEM analysis. The ceria nanoparticle with 45.8% Ce³⁺ are slightly oxidized to have 41.3% of Ce³⁺ after the reaction with Mn²⁺ as evidenced by XPS (FIG. 3).

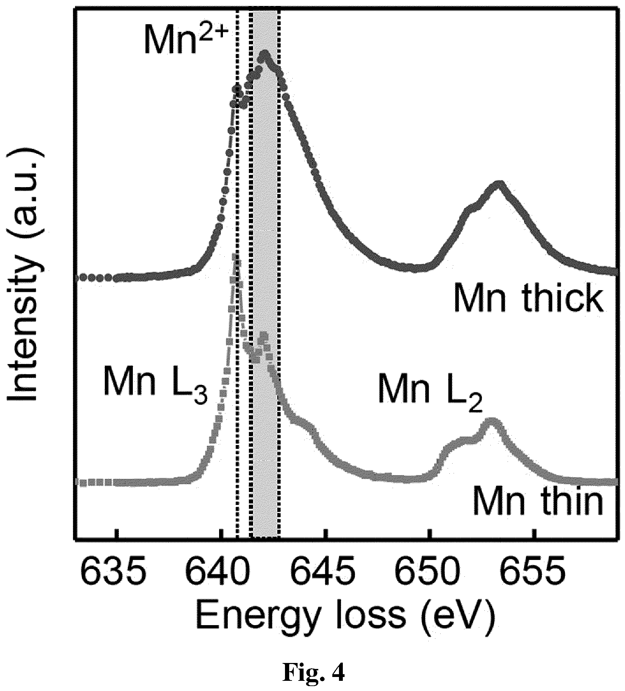

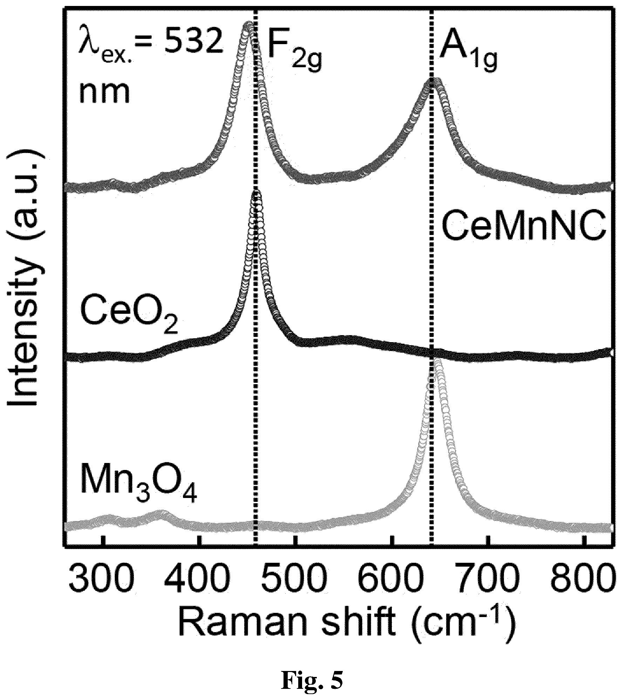

When less than 15% of Mn compared to Ce is deposited on the ceria nanoparticles, the oxidation state of Mn is mostly Mn²⁺ and Ce⁴⁺-O-Mn²⁺ polar interface is formed (FIG. 4) . However, in 1 to 3 atomic layers of manganese oxide, both Mn²⁺ and Mn³⁺ are observed. Combining the XPS, XAS data, and known lattice parameter and atomic arrangements of Mn₃O₄, the heterostructured cerium-manganese oxide nanoparticle (CeMnNC) is composed of CeO-₂, Ce⁴⁺-O-Mn²⁺ pairing at the heterointerface and Mn₃O₄. This is supported by visible (532 nm) Raman spectra (FIG. 5). The spectra of cerium-manganese oxide nanoparticle show both first-order F_{2g} symmetry mode of CeO₂ (452 cw¹) and A_{1g} symmetric stretching Mn-O bond peak of Mn₃O₄ (645 cm⁻¹). Broadening of the peaks can be explained by symmetry lowering of Ce-O bond and Mn-O bond at the heterointerface between CeO₂ and Mn₃O₄. Red shift and asymmetric peak broadening of F_{2g} peak indicate a higher number of defects that are essential for scavenging ROS.

The broadened A_{1g} peak is originated from an epitaxial strain in Mn₃O₄ layer induced by the polar interface (Ce⁴⁺-O-Mn²⁺) and lattice mismatch. The morphology of Mn₃O₄ layer can also support the epitaxial strain. Since lattice mismatch between CeO₂ (111) plane and Mn₃O₄ (112) plane is only about 1.2%, Mn₃O₄ can grow in [112] direction parallel to CeO₂ (111) plane.

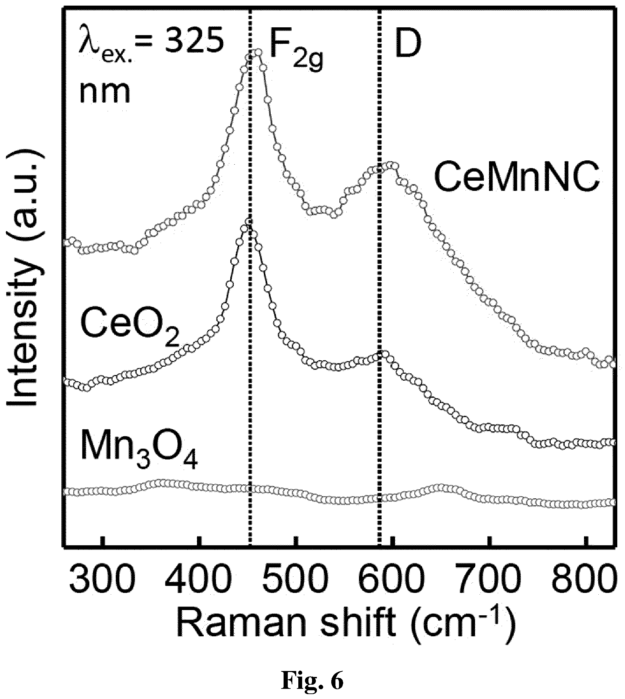

To minimize the strain induced from large lattice mismatch along other planes, the high-index facet of Mn₃O₄ {332}, which is the step of {112} plane, can be exposed (FIG. 2). The UV (325 nm) Raman spectra are used to compare the surface defects between CeO₂ nanoparticle and cerium-manganese oxide nanoparticle (FIG. 6). The intensity ratio (I_{D}/I_{F2g}) of defect-induced (D mode) peak at 598 cm⁻¹ and F_{2g} peak is increased after the Mn₃O₄ deposition, which means the increased O-vacancies on the CeO₂ surface.

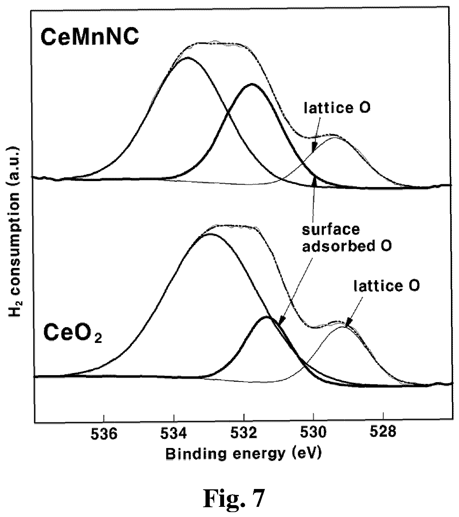

The cerium-manganese oxide nanoparticles with abundant O-vacancies exhibit high activity for ROS scavenging, which can be attributed to charge delocalization over the entire nanoparticles, though the cerium-manganese multimetallic oxide nanoparticles have a slightly smaller concentration of Ce³⁺ than that of CeO₂. (There are no Ce³⁺ sites in chemically active colloidal ceria nanoparticle). Defective oxide surfaces also can be detected by O Is XPS spectra (FIG. 7).

Binding energies of about 529.2 eV, about 531.5 eV and about 533.2 eV can be assigned to lattice oxygen (Oₗₐₜₜ), surface adsorbed oxygen (O_{ads}) and chemisorbed species (O_{surf}) such as water, respectively. Since the range of 531∼532 eV represents the defective sites having electrophilic O₂²⁻, O²⁻, and O⁻ on the surface, the oxygen defects concentration can be estimated from O_{ads}/Oₗₐₜₜ ratios.

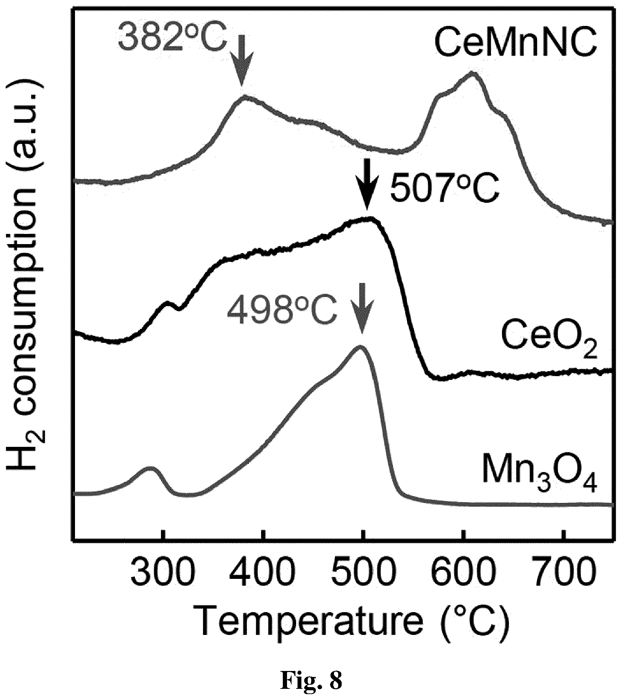

The higher ratio of cerium-manganese oxide nanoparticle (2.16) than that of CeO₂ (1.04) reveals the higher oxygen vacancy level in cerium-manganese oxide nanoparticles, in good agreement with what is obtained from Raman analysis. To analyze the surface reducibility, H₂ TPR (temperature-programmed reduction) is measured (FIG. 8). Both surface oxygen reduction peak at 507 °C for CeO₂ and Mn₃O₄ reduction peak of Mn²⁺ at 498 °C are shifted to a lower temperature (382 °C), and this indicates the enhancement of surface reducibility for the cerium-manganese oxide nanoparticle.

According to TEM, XPS, XAS, Raman and H₂ TPR results, a few layers of Mn₃O₄ in addition to the Ce⁴⁺-O-Mn²⁺ polar interface produced by the heteroepitaxy process are necessary to generate the high redox-active surface of cerium-manganese oxide nanoparticle.

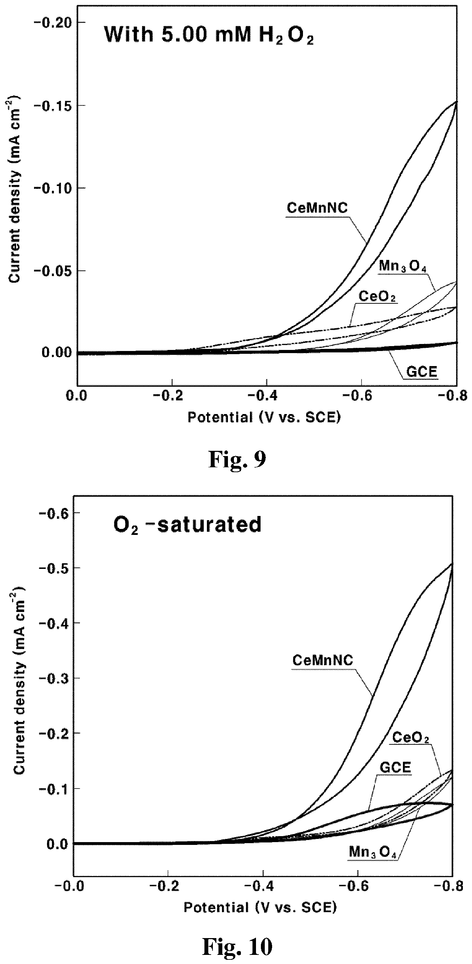

FIGS. 9 and 10 show the catalytic activities of CeO₂, cerium-manganese oxide nanoparticle and Mn₃O₄ toward the reduction of H₂O₂ and oxygen. FIGS. 9 and 10 show cyclic voltammetric (CV) scans of three samples supported on glassy carbon electrodes (GCE) in Ar-saturated phosphate-buffered saline (PBS, 0.01 M) in the presence of 5.00 mM H₂O₂ and in O₂-saturated PBS, respectively. The cerium-manganese oxide nanoparticle shows the sharp increase of reduction current under -0.4 V, and this means improved catalytic activities when compared to monometallic CeO₂ and Mn₃O₄.

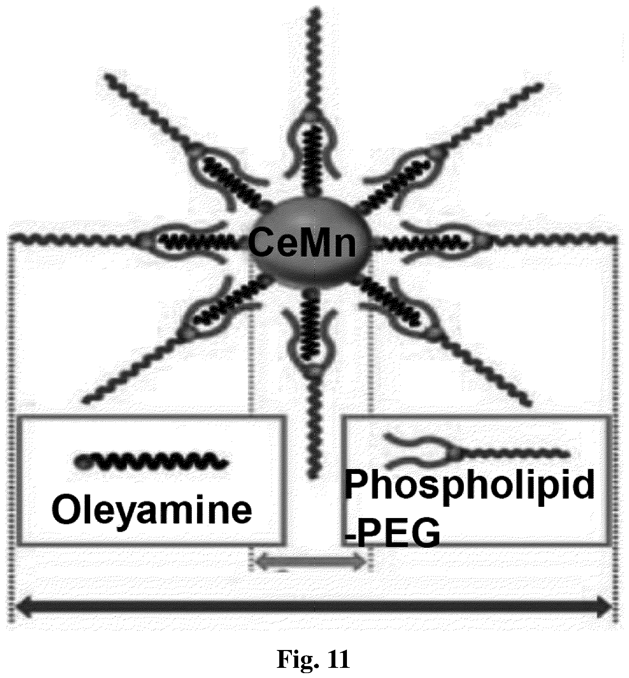

FIGS. 11 to 17 are views for explaining the enzyme mimetic activity of cerium-manganese oxide nanoparticle.

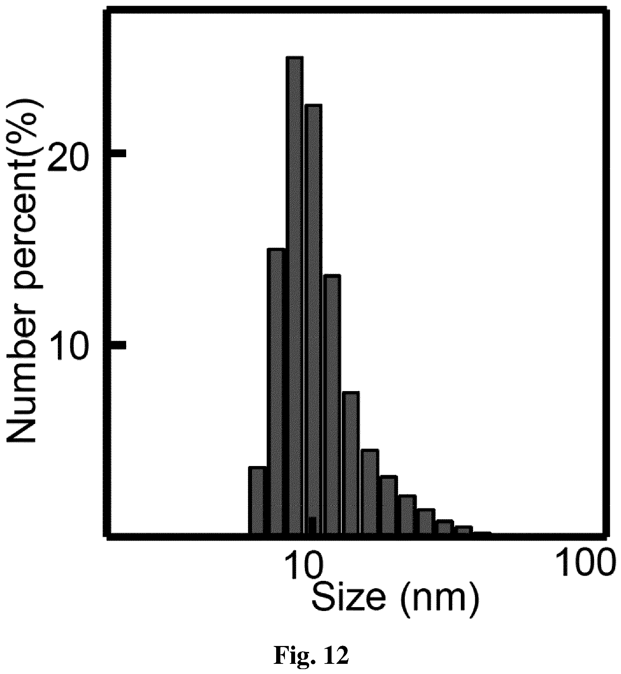

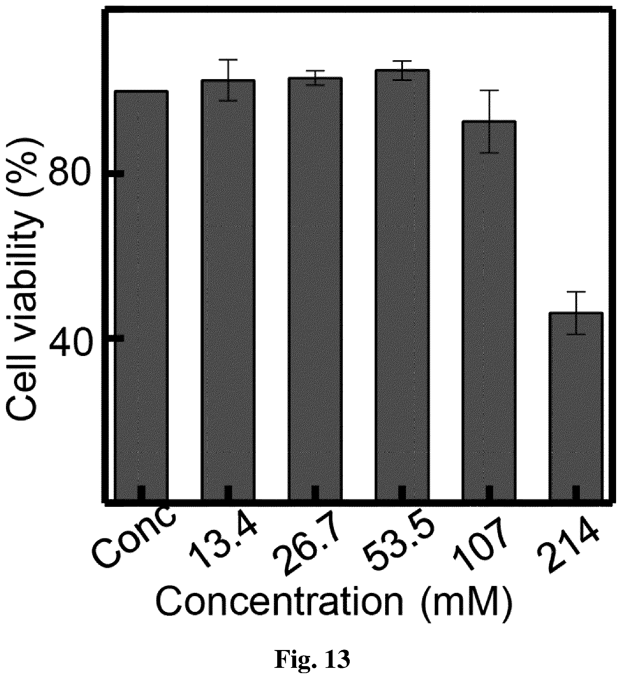

Cerium-manganese oxide nanoparticles are transferred into the water-dispersible hydrophilic nanoparticles using PEGylation method (FIG. 11). The PEGylated cerium-manganese oxide nanoparticles exhibit hydrodynamic diameter ranging from 9 to 15 nm in PBS (phosphate-buffered saline) (FIG. 12). An analysis of cell viability according to the concentration of the cerium-manganese oxide nanoparticles shows that the cerium-manganese oxide nanoparticles are excellent in biocompatibility (FIG. 13).

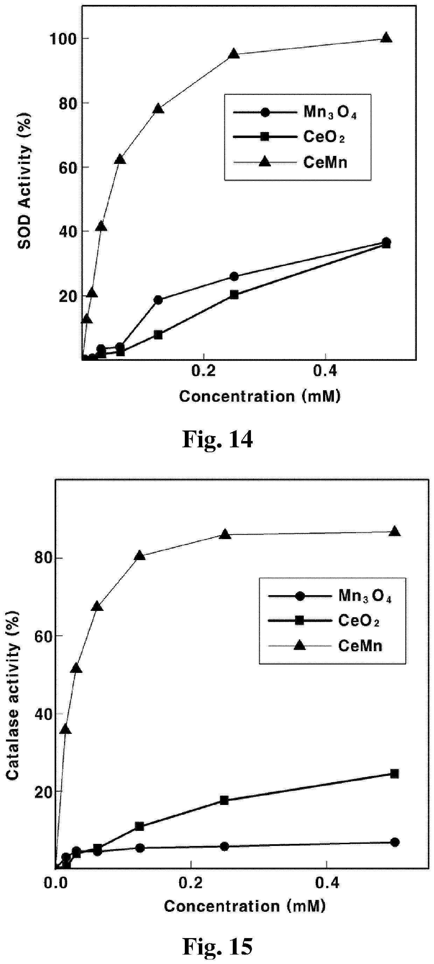

The enzymatic activity of ceria nanoparticles (CeO₂) and cerium-manganese oxide nanoparticles (CeMn) in aqueous media is demonstrated for three representative ROS, O²⁻, H₂O₂, •OH using SOD, CAT mimetic and HORAC (hydroxyl radical antioxidant capacity) assays (FIGS. 14 to 16).

The cerium-manganese oxide nanoparticles (CeMn) exhibit outstanding enzymatic performance in all of three representative ROS when compared to CeO₂ or Mn₃O₄ nanoparticles. This is due to fact that Mn ions introduced into CeO₂ nanoparticles increase oxygen vacancies on CeO₂ surface and reducibility of cerium-manganese oxide nanoparticles. Among them cerium-manganese oxide nanoparticles with thick Mn layers exhibit better enzymatic performance than thinner Mn layers since exposing high index facet Mn {332} provides highly catalytically active site.

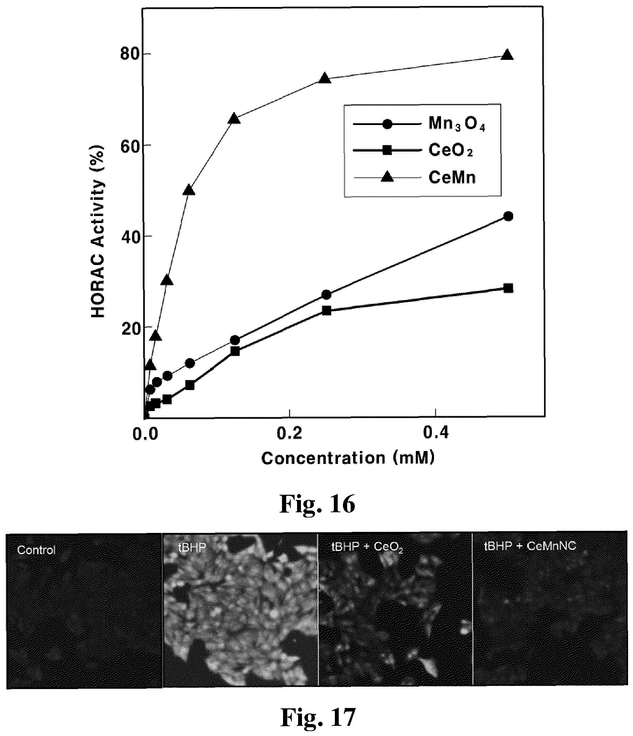

To analyze ROS scavenging capability of the cerium-manganese oxide nanoparticles, fluorescence probe is used to measure intracellular levels of ROS (FIG. 17). Strong fluorescence signal is observed in cells treated with tBHP alone, and the ROS level is greatly enhanced in comparison with the control. However, this fluorescence signal is greatly inhibited when tBHP is co-treated with CeO₂ and further inhibited when treated with cerium-manganese oxide nanoparticles, and thus both CeO₂ nanoparticles and cerium-manganese oxide nanoparticles exhibit antioxidant capability but cerium-manganese oxide nanoparticles exhibit better scavenging ability in cellular level when comparing with CeO₂ nanoparticles.

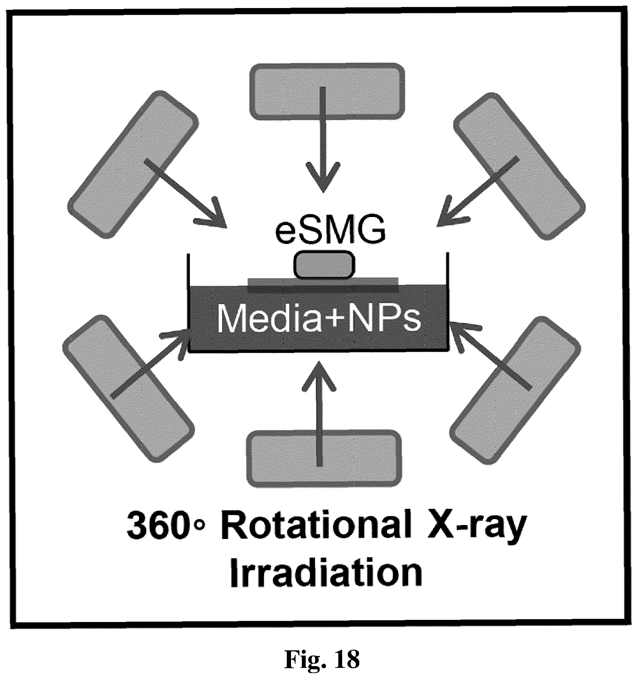

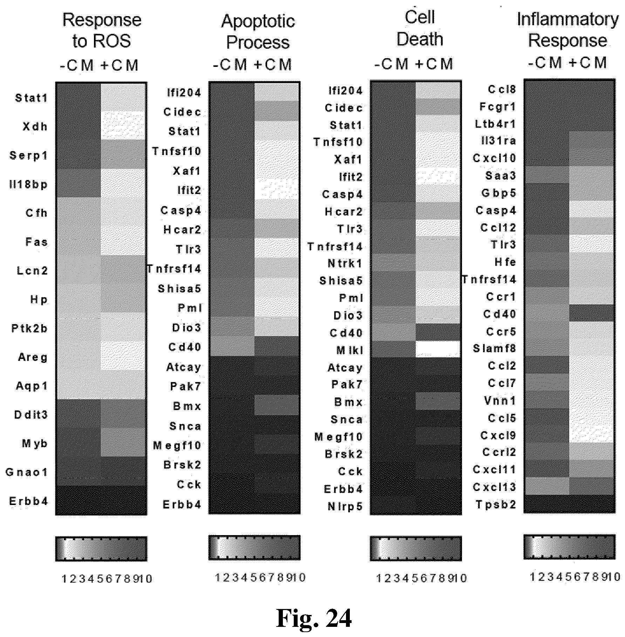

FIGS. 18 to 24 are views for explaining the protection of cerium-manganese oxide nanoparticle for eSMG from radiation induced damages.

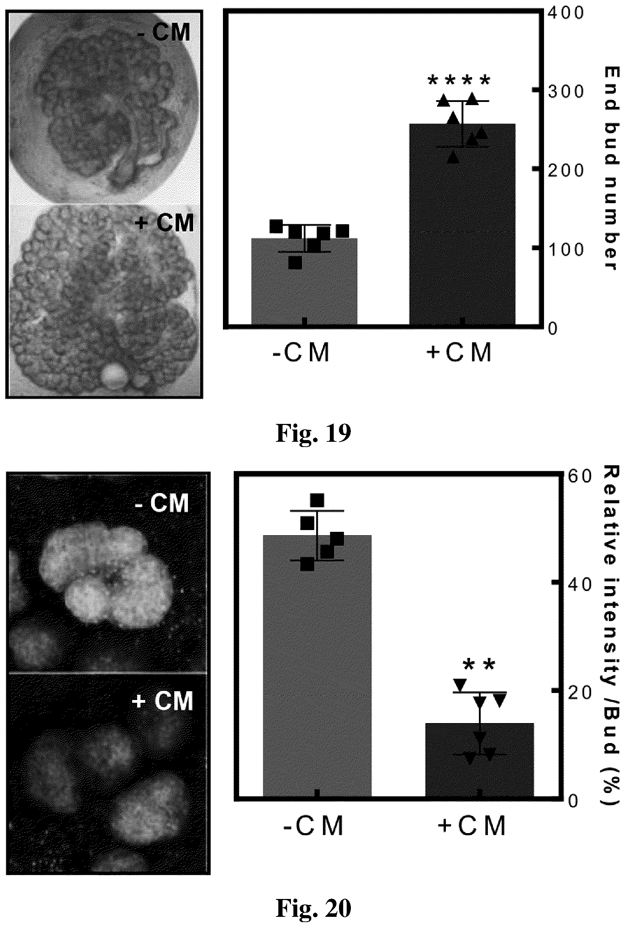

In order to examine radioprotective ability of the cerium-manganese oxide nanoparticle (CeMn) for serum-free embryonic submandibular gland (eSMG), organoid ex-vivo culture model having many advantages over other models is used. For example, in vitro model is unable to reflect interactive relationships among multiple cell types, and in vivo salivary gland IR damage model is highly time and resource-consuming. However eSMG model requires only 48∼72 hours to examine IR-induced damages while maintaining complex biological interactions among various cell types including progenitor cells, vascular endothelial (VE) cells, epithelial acinar/ductal cells, mesenchymal cells and parasympathetic ganglion cells (FIGS. 18 and 19).

Level of intracellular ROS is measured via CellRox Green and ROS level within the epithelial buds shows significantly lower in cerium-manganese oxide nanoparticle treated group in comparison with untreated group. This indicates that the cerium-manganese oxide nanoparticle effectively scavenges ROS generated by IR (FIG. 20). C-kit+ salivary gland progenitor cells are maintaining their survivals and self-renewal capacity by interacting with surrounding microenvironments or niche such as mesenchymal cells, parasympathetic ganglion (PSG) and vascular endothelial (VE) cells.

When salivary gland acinar cells are damaged c-kit+ progenitor cells regain their activity from dormancy to proliferate and differentiate into new acinar cells. Therefore protecting progenitor cells and their niche from radiation damages is crucial for the salivary gland regeneration.

Therefore, immuno-histologic analysis is performed to see the radioprotective effect of the cerium-manganese oxide nanoparticle on epithelial acinar/ductal cells, VE cells, PSG and c-kit+ progenitor cells. Acinar/ductal cell ratio is frequently disrupted in irradiated salivary glands in human and this phenomenon is also observed in mouse eSMG models. eSMGs irradiated with 13 Gy show significantly decreased acinar (AQP5+) /ductal (K19+) cell ratio, but cerium-manganese oxide nanoparticle-treated group shows improved acinar/ductal cell balance.

PSG growth and branching are significantly inhibited in +IR/-CM group but +IR/+CM group manages to recover PSG growth up to normal levels. Apoptotic rate of whole explants is measured by Caspase3/7 Glo assay. +IR/-CM group shows significantly increased caspase3/7 activities than +IR/+CM, this indicates that cerium-manganese oxide nanoparticles effectively protect from IR induced apoptosis.

Apoptotic rate of acinar cells, PSG and c-kit+ progenitor cells is measured by counting active caspase 3-positive cells in each cell types. In all three types of cells, +IR/+CM group shows significantly less apoptotic rate in comparison with +IR/- CM group. Number of c-kit+ progenitor cells is drastically decreased in +IR/-CM group, but c-kit+ cell population is well-preserved in +IR/+CM group.

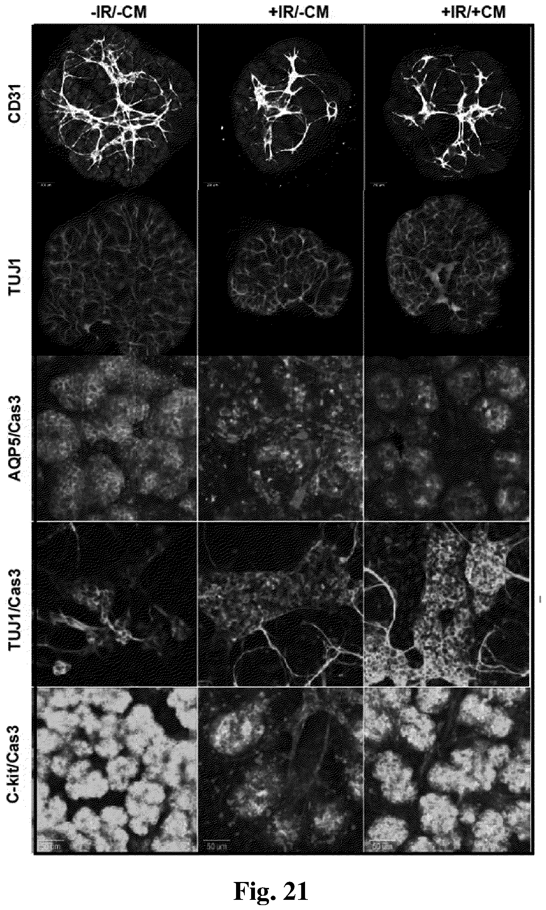

These survived c-kit+ progenitor cells in +IR/+CM group is later successfully differentiated into AQP5+ acinar cells, but +IR/-CM group shows severely decreased number of differentiated acinar cells (FIG. 21).

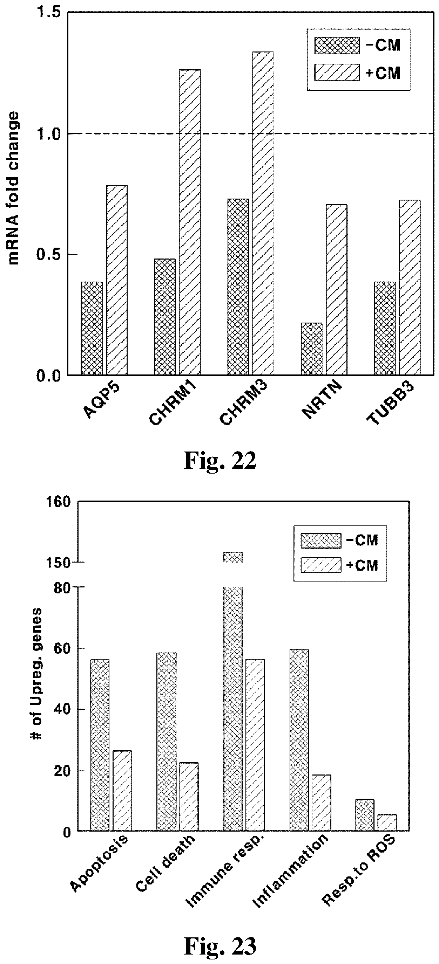

The protection efficacy of the cerium-manganese oxide nanoparticle is further confirmed quantitatively by mRNA expression levels for acinar cells (AQP5, CHRM1, CHRM3), PSG (TUJ1, NRTN) and c-kit+ progenitor cells (C-kit) (FIGS. 22 to 24) .

Expression levels of all markers in cerium-manganese oxide nanoparticle-treated eSMGs group are higher than those in untreated group, which is similar to the immunofluorescence data. These results indicate that cerium-manganese oxide nanoparticles provide global radio-protection to progenitor cells and their surrounding structures, which helps progenitor cells to survive, proliferate and differentiate into functional acinar cells.

Global gene expression levels of each group (mRNA extracted from 10 eSMGs per group) are compared via Next Generation Sequencing in order to investigate a global vicious cycle of biological events occurring after IR. Increased ROS by IR may induce immune responses, inflammation and apoptotic cell death. Cell debris from dead cells may cause further increased inflammation and ROS level may induce more cell death. IR evokes the dramatic increase of various genes related to ROS, inflammation response, immune response and apoptosis, but cerium-manganese oxide nanoparticle treatment mitigates the expression levels of such genes. Although eSMG organoid models do not have circulating immune cells, they partially reflect acute inflammations and immune responses induced by IR, and biological processes involved in a radiation-induced vicious cycle are also successfully blocked by the cerium-manganese oxide nanoparticle.

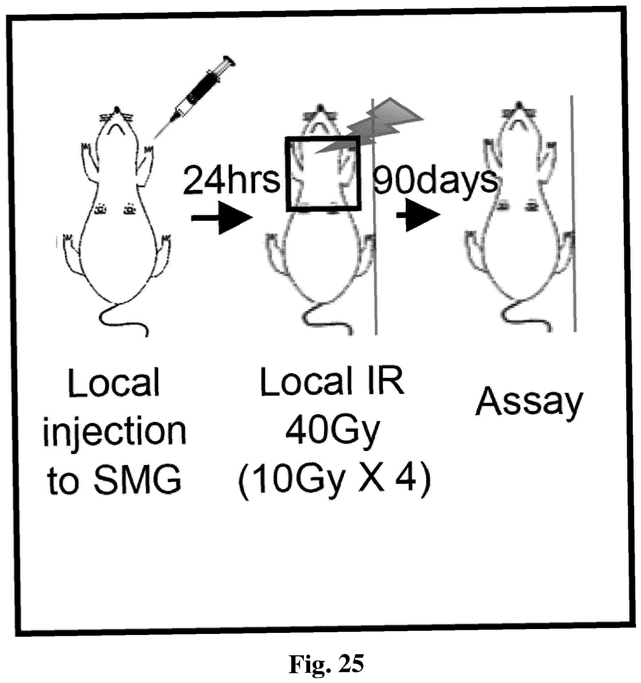

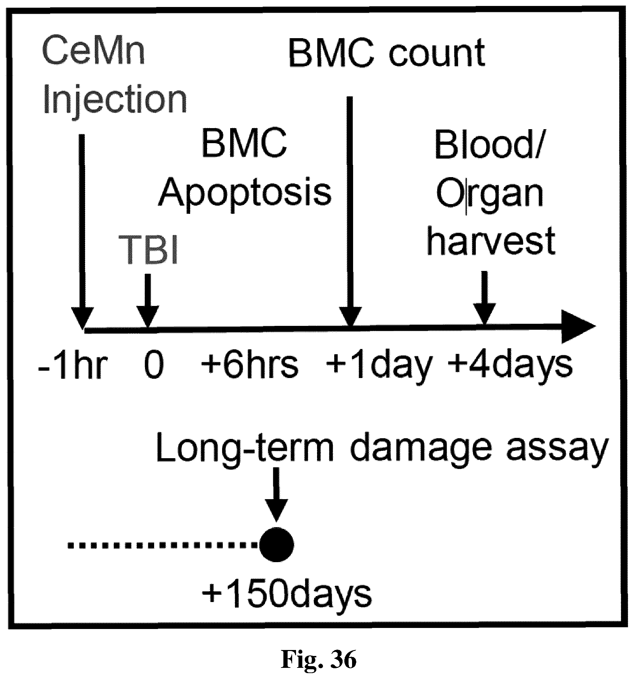

FIGS. 25 to 35 are views for explaining the protection of cerium-manganese oxide nanoparticle against IR locally irradiated in vivo, and FIGS. 36 to 49 are views for explaining the protection of cerium-manganese oxide nanoparticle against IR entirely irradiated in vivo.

CeMn (cerium-manganese oxide nanoparticle)'s ability to protect structure and functions of SMG in vivo is tested. Furthermore, oral and systemic health from IR-induced Xerostomia is tested. Cerium-manganese oxide nanoparticles are injected into both SMGs of ICR mice through each duct before IR. The locations of SMGs are visually identified and X-ray irradiation is conducted with the dose of 5 Gy per day for 4 consecutive days (FIG. 25).

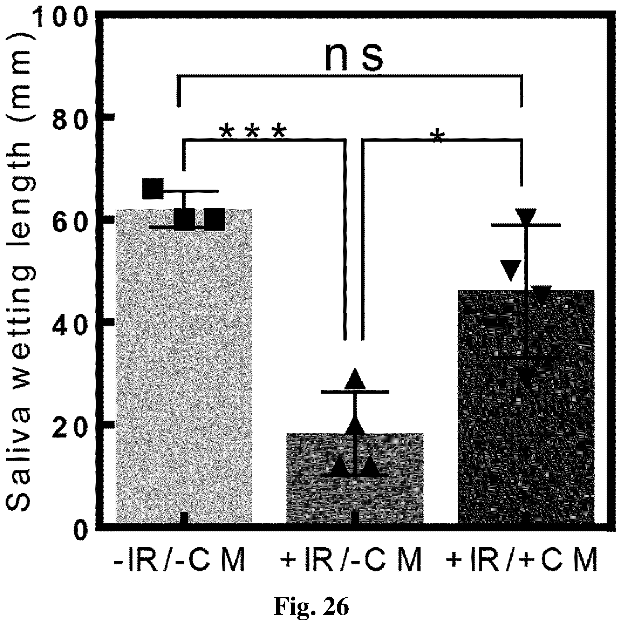

90 days later mice are subject to salivary gland function tests and histological analysis. Salivary gland function test is performed by collecting whole saliva from mice stimulated with pilocarpine. After IR, average salivary flow rate is decreased from 3.5 µl/min to 1.2 µl/min, but cerium-manganese oxide nanoparticle-treated group shows significantly improved salivary flow rate of 2.5 µl/min (FIG. 26).

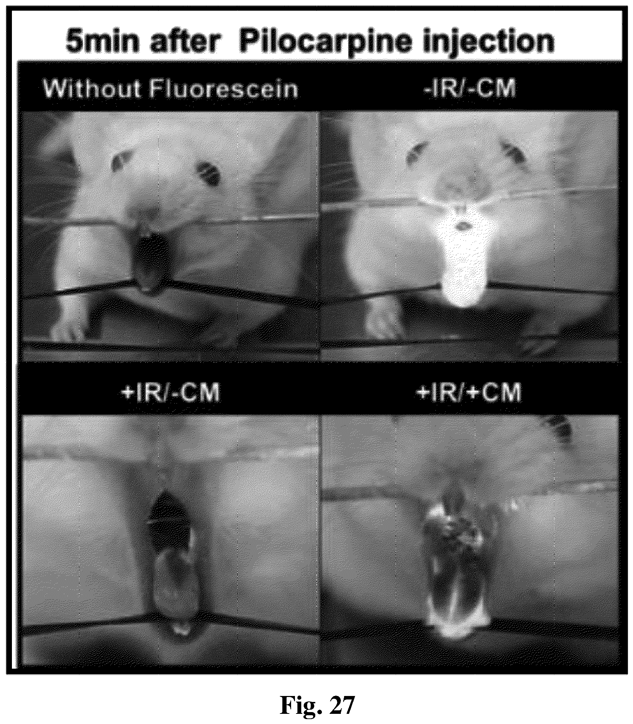

Since it is important for saliva to fully cover oral tissues such as tongue or buccal mucosa, the area of oral cavity covered by produced saliva is measured with sodium fluorescein paper (FIG. 27). Almost no fluorescein dye is observed within oral cavities of +IR/-CM group, but in +IR/+CM group fluorescein dye is found on ventral and dorsal sides of tongue, palate and buccal mucosa.

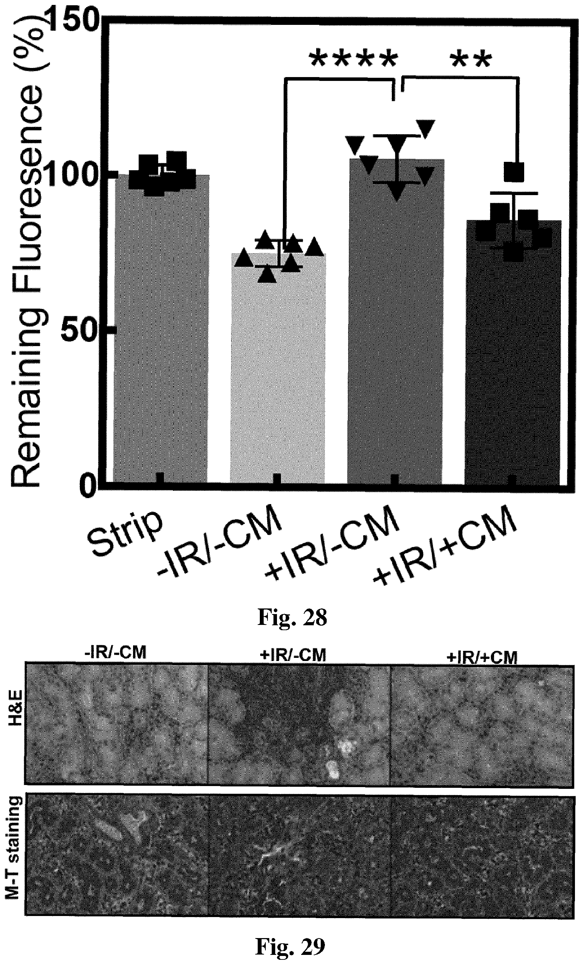

Amount of fluorescein dye left on the removed papers is confirmed by spectrophotometer. About 20% of dye is washed off by saliva in -IR/-CM and +IR/+CM groups but almost no dye is diffused out in +IR/-CM group (FIG. 28).

SMGs' histopathological changes induced by IR are examined (FIG. 29). Considerable increase in leukocyte infiltration and tissue fibrosis is observed in +IR/-CM group. However, +IR/+CM group shows no significant difference in leukocyte infiltration or fibrotic change in comparison with -IR/-CM group.

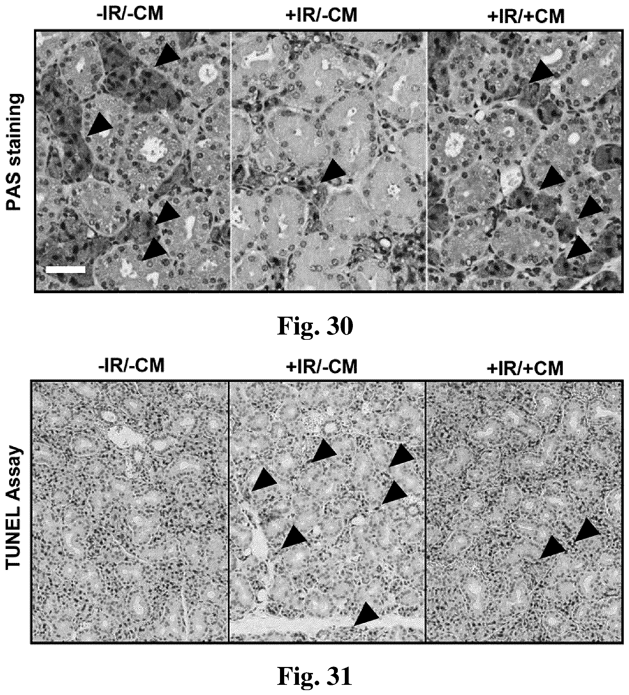

Secretory acinar cells are visualized with PAS staining (FIG. 30). Acinar cells are barely observed in +IR/-CM group, but significantly increased number of acinar cells are observed in +IR/+CM. Area of acinar/ductal cells is quantified and as observed in eSMG IR model, +IR/-CM group shows significantly decreased acinar/ductal ratio.

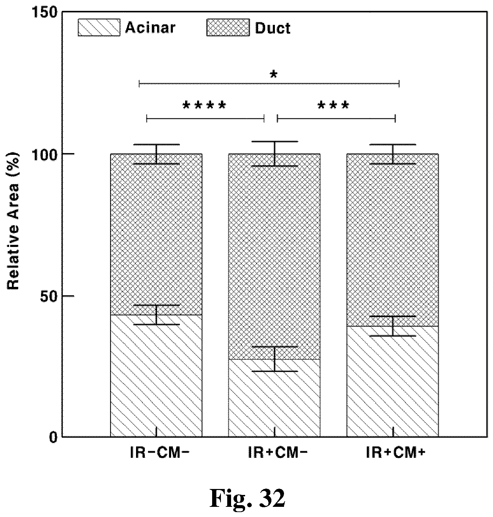

However disrupted acinar/ductal cell ratio is partially restored in +IR/+CM group. Actual apoptosis of SMG tissue is examined by TUNEL assay, and as presented cerium-manganese oxide nanoparticle treated group shows significantly decreased TUNEL+ cell count (FIGS. 31 and 32). Radiation-induced Xerostomia causes inflammations in oral tissues, loss of taste, disruption of oral microflora and dysphagia, which make patients difficult to eat foods.

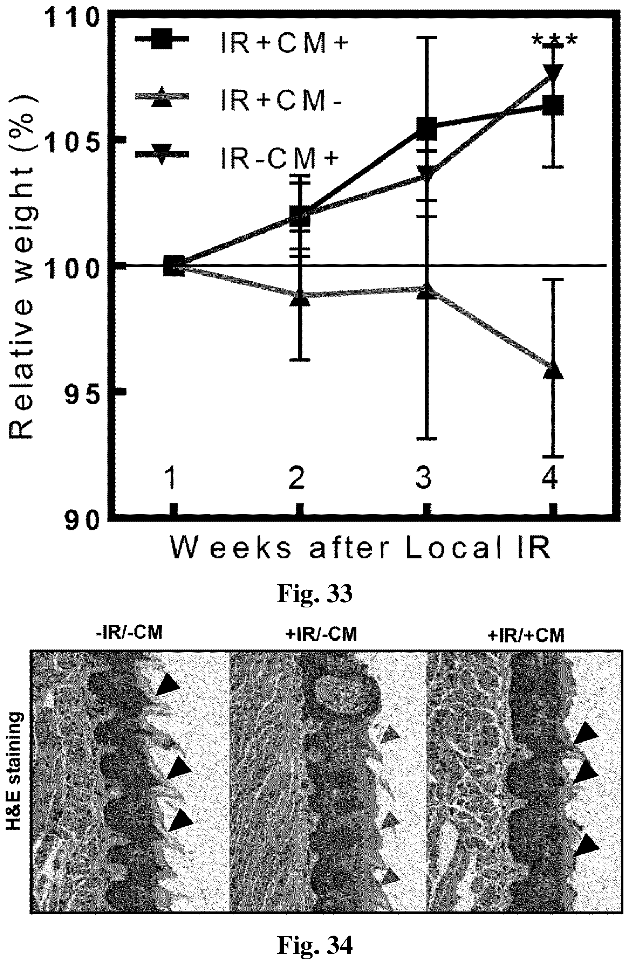

Localized IR to SMGs leads to significant loss of body weight at 4 weeks after IR, which is similar to clinical symptoms. However, cerium-manganese oxide nanoparticle-treated mice show no significant weight loss (FIG. 33). The tongue of IR mice shows blunt-shaped filiform papilla and thickened cornified layers, but tongues of cerium-manganese oxide nanoparticle-treated group show normal thickness of cornified layers and coexistence of normal and blunt shaped filiform papilla (FIG. 34) .

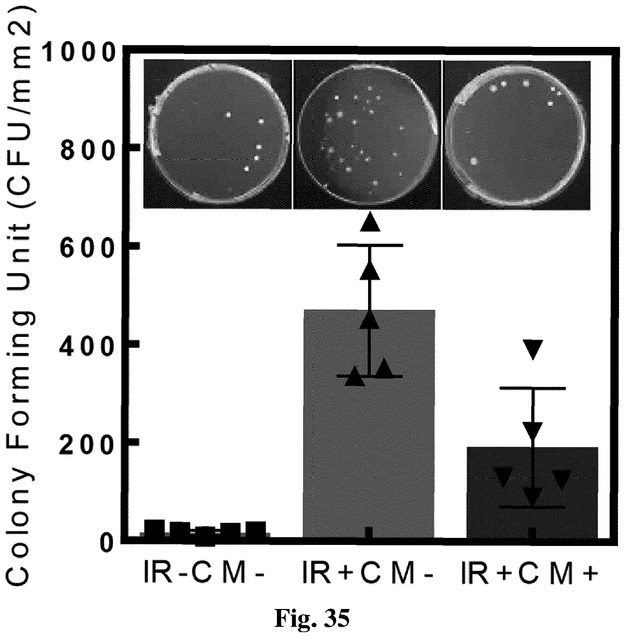

Anti-microbial activity of saliva is one of the most important functions in saliva. Oral bacteria samples are collected by buccal swap and cultured for the colony forming assay. Abnormally increased number of bacteria colony is observed in +IR/-CM group but it is partially significantly decreased in +IR/+CM group (FIG. 35). These results indicate that locally injected cerium-manganese oxide nanoparticles can protect salivary glands' structure and function and thus prevent various oral and systemic complications induced by salivary gland IR.

Mice are intraperitoneally injected either with PBS or 50mg/kg of cerium-manganese oxide nanoparticles, and body weights are measured in 3-days interval for 30 days. There is no significant loss of body weight in cerium-manganese oxide nanoparticle-injected group in comparison with PBS-injected group. After 30 days mice are sacrificed and organs are harvested for the histopathological examination. Also no significant pathological change is observed for cerium-manganese oxide nanoparticle treated mice in 7 major organs including intestine, kidney, spleen, liver, heart, lung and bladder. This indicates that cerium-manganese oxide nanoparticles do not have notable systemic toxicity. CeMn's systemic radio-protection ability under 0.55 mg/kg concentration which is 1/100 of cerium-manganese oxide nanoparticle concentration used in toxicity test is tested.

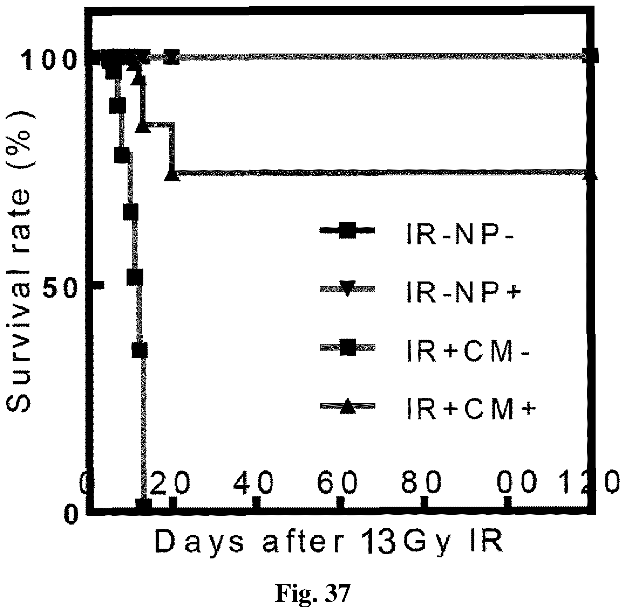

Mice are subjected to total body irradiation (TBI) with 13 Gy after the injection of cerium-manganese oxide nanoparticles (FIG. 36). No mouse dies in -IR/-CM and -IR/+CM groups, and this proves that active dose of cerium-manganese oxide nanoparticles does not cause any systemic toxicity. Survival rate of mice in +IR/-CM group is drastically decreased to 50% at 12 days post-TBI and at the point of 15 days after TBI, all of 15 mice are dead. However, 10 mice survive in +IR/+CM group at the 150 days after TBI (FIG. 37).

In order to figure out the factors contributing to the increased survival of cerium-manganese oxide nanoparticle treated group, short and long term pathophysiological changes of blood, bone marrow cells (BMC) and small intestine that are the organs largely damaged by Acute Radiation Syndrome (ARS) are observed. It has been known that the most acute response to the radiation exposure is elevation of cytokine in circulatory system, which may cause nausea and fatigue.

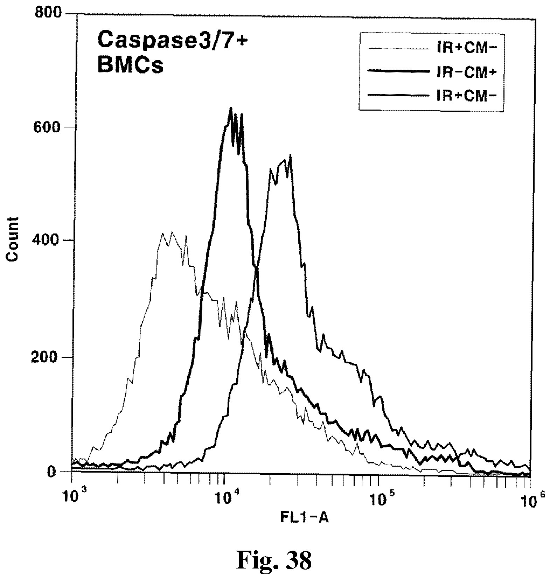

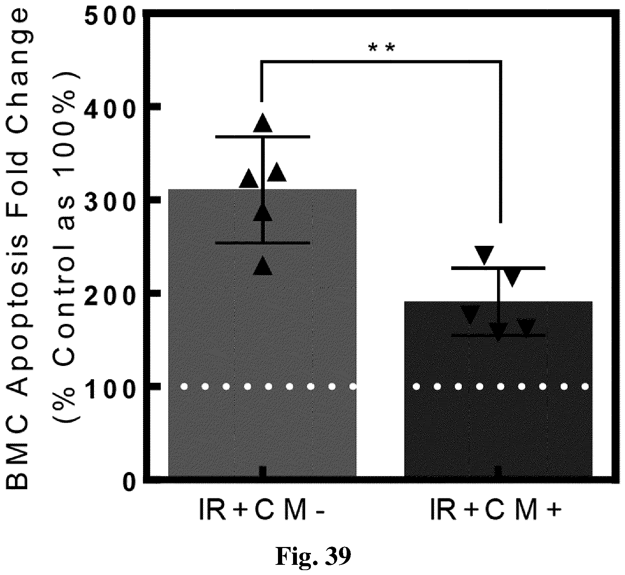

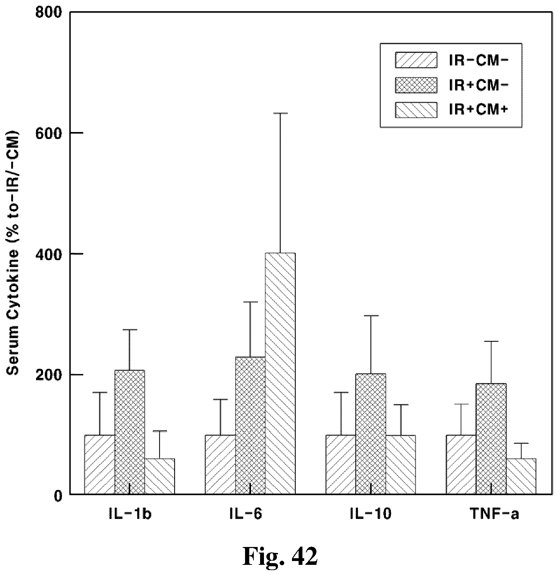

6 hours after TBI, serum levels of four blood cytokines including Interleukin-lbeta (IL-1b), Interleukin-6 (IL-6), Interleukin-10 (IL-10) and Tumor Necrosis Factor-a (TNF-a) are increased (FIG. 42). However, cerium-manganese oxide nanoparticle-treated group shows significantly less elevated four cytokine levels than cerium-manganese oxide nanoparticle-untreated group. Mouse BMC apoptotic rate is measured after TBI and BMC apoptosis is significantly inhibited in cerium-manganese oxide nanoparticle-treated group (FIGS. 38 and 39).

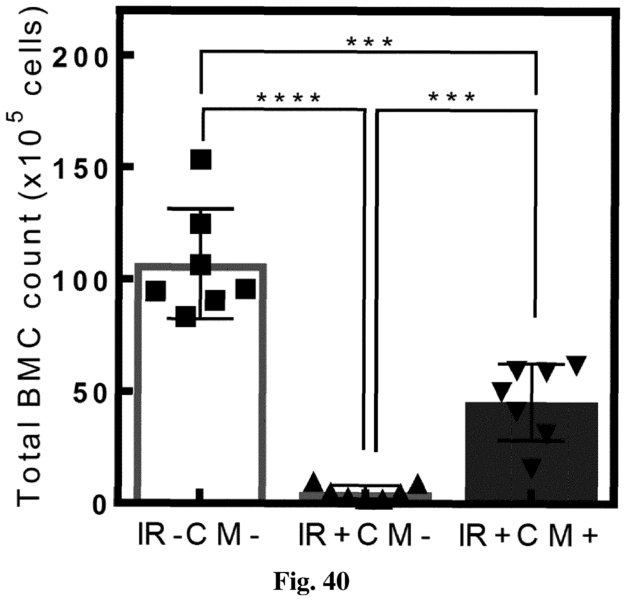

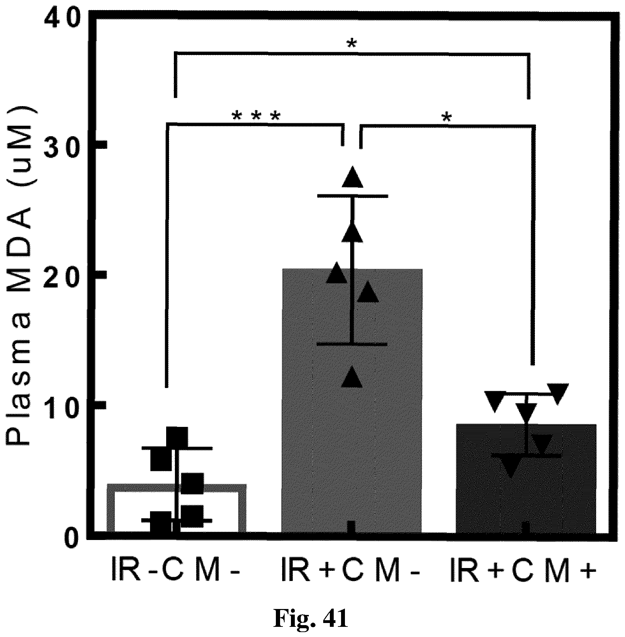

One day after TBI total number of BMC is also isolated and counted (FIG. 40). Almost 95% of BMCs are ablated in cerium-manganese oxide nanoparticle-untreated group but only average of 50% of BMCs is ablated in cerium-manganese oxide nanoparticle-treated group. Plasma MDA level is measured one day after TBI and CeMn-treated group shows significantly decreased MDA concentration in plasma than CeMn-untreated group (FIG. 41).

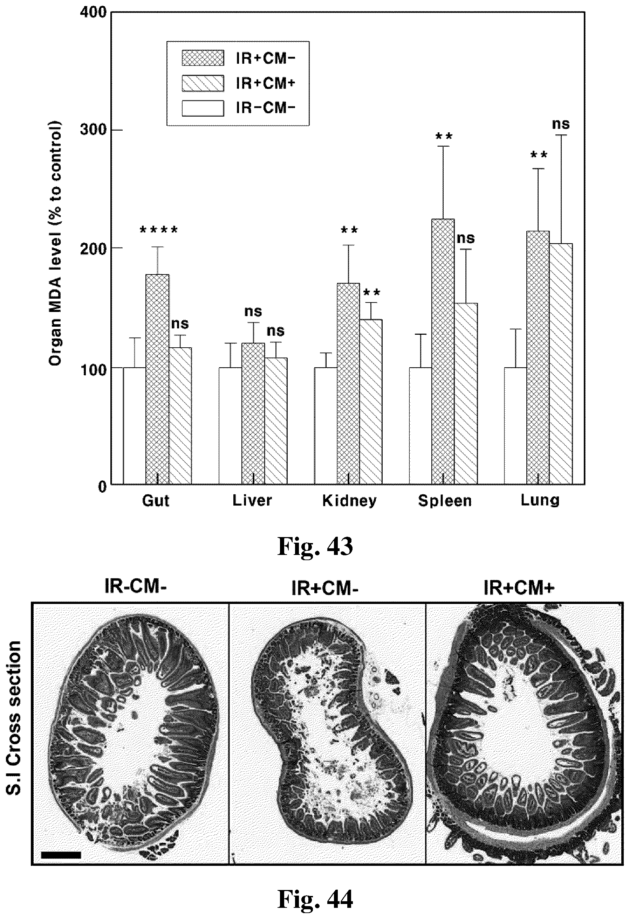

Four days after TBI, MDA levels of 5 organs including small intestine, liver, kidney, spleen and lung are measured (FIG. 43). Except for liver, all other organs show significantly increased MDA level after the TBI. In comparison with -IR/-CM group no significant elevation of MDA level is observed in small intestine, spleen and lung of cerium-manganese oxide nanoparticle-treated group.

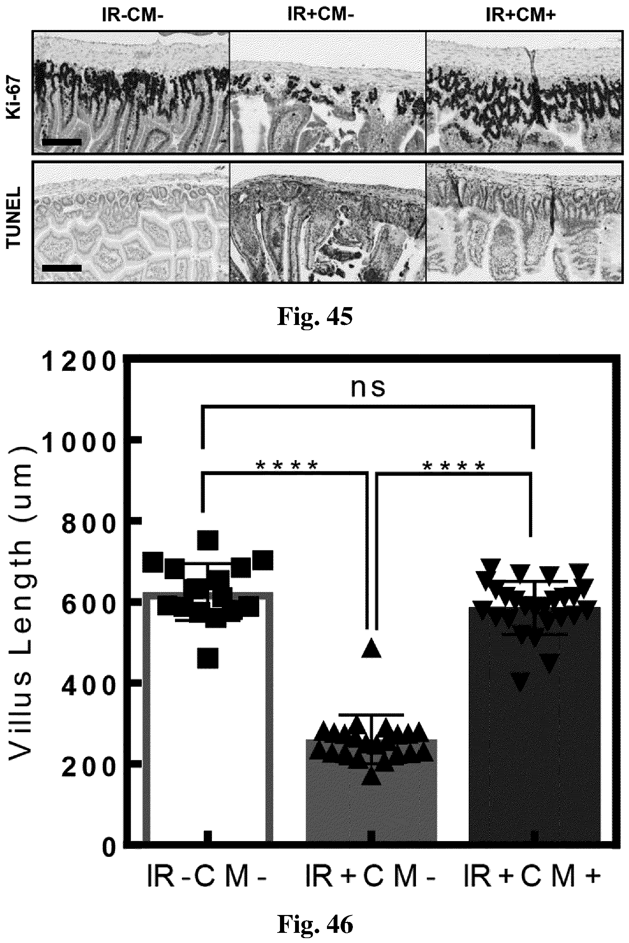

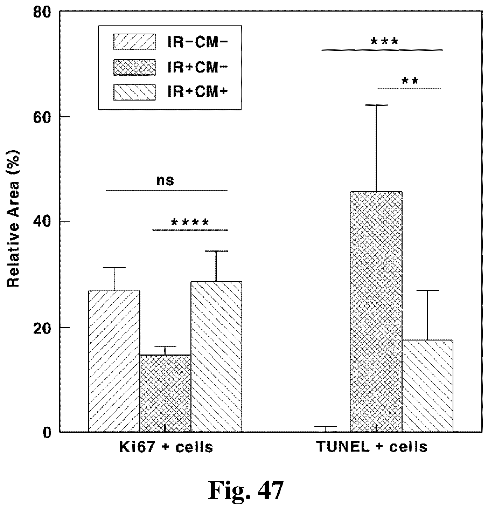

The radiation-induced small intestine damages which may cause radiation enteropathy are examined (FIG. 44). One week after TBI, duodenums are harvested and histopathological assays are conducted. Integrities of villus are severely damaged by TBI, but cerium-manganese oxide nanoparticle-treated group shows relatively intact villus structure and preserves longer villus length than cerium-manganese oxide nanoparticle-untreated group (FIGS. 46 and 47).

TUNEL assay and Ki-67 staining results also indicate that cerium-manganese oxide nanoparticles can protect both villus and crypt cells from IR, and maintain mitotic activity of stem and progenitor cells in crypts (FIG. 45).

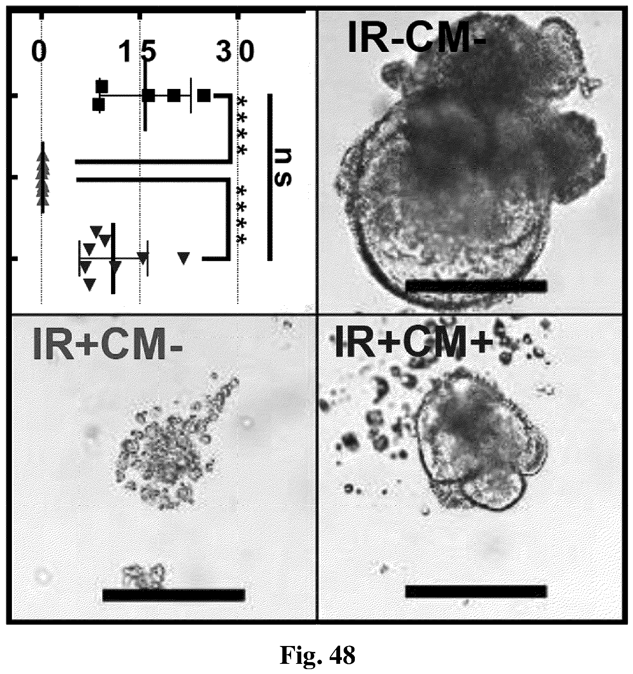

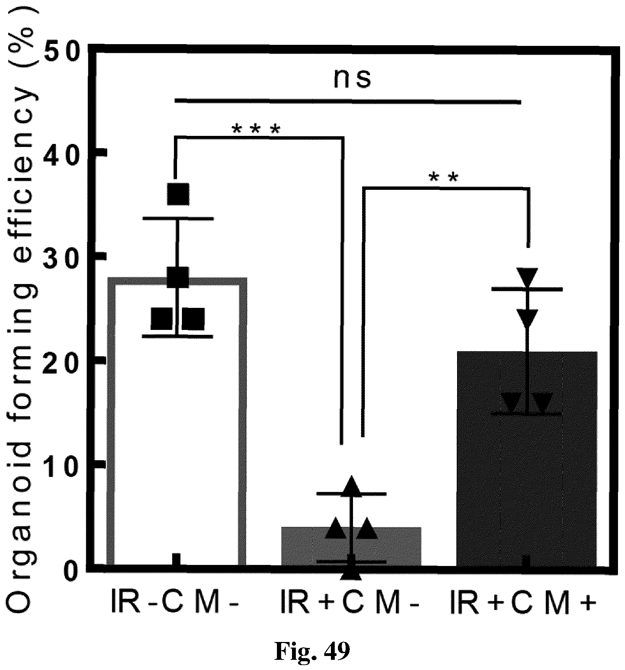

In order to confirm self-renewal ability of these survived crypt cells for cerium-manganese oxide nanoparticle-treated group, crypts from mouse within 30 min after TBI are isolated and cultured in in-vitro organoid culture condition (FIG. 48). After 7 days crypts isolated from cerium-manganese oxide nanoparticle-untreated group are unable to form intestinal organoids but cerium-manganese oxide nanoparticle-treated group shows significantly increased organoid forming efficiency (FIG. 49). Also intestinal organoids from cerium-manganese oxide nanoparticle-treated group present normal organoid phenotypic characteristics including central lumen, crypt structure and villus. However, crypts isolated from cerium-manganese oxide nanoparticle-untreated group cannot even form spherical cyst for 7 days of culture.

Finally, long-term damage assessment by harvesting organs from survived cerium-manganese oxide nanoparticle-treated mice 150 days after TBI is conducted. Both duodenum and jejunum of survivors show no sign of tumorigenesis or pathogenesis.

Although the embodiments of the present invention have been disclosed for illustrative purposes, those skilled in the art will appreciate that the present invention may be embodied in other specific ways without changing the technical spirit or essential features thereof. Therefore, the embodiments disclosed in the present invention are not restrictive but are illustrative. The scope of the present invention is given by the claims, rather than the specification, and also contains all modifications within the meaning and range equivalent to the claims.

### Industrial Applicability

A nanoparticle for radiation protection according to the embodiments of the present invention can have excellent radiation protection ability and biocompatibility. The nanoparticle for radiation protection can increase the ability of scavenging reactive oxygen species and reduce systemic toxicity. In addition, the nanoparticle for radiation protection can have long-lasting stable activity and low active dose. The nanoparticle for radiation protection can be used locally and systemically to protect various radiation-induced damages. The nanoparticle for radiation protection is non-toxic or has low toxicity, and it can also directly protect tissues and cells from radiation with small dose, thereby promoting faster regeneration and recovery of functions.

## Claims

1. A nanoparticle for radiation protection comprising a first metal oxide nanoparticle.

2. The nanoparticle for radiation protection of claim 1, further comprising a second metal oxide layer formed on a surface of the first metal oxide nanoparticle.

3. The nanoparticle for radiation protection of claim 2, having a polar interface between the first metal oxide nanoparticle and the second metal oxide layer.

4. The nanoparticle for radiation protection of claim 3, wherein the polar interface comprises (a metal ion of the first metal oxide)-O-(a metal ion of the second metal oxide) pairing.

5. The nanoparticle for radiation protection of claim 2, wherein the second metal oxide layer is formed on the surface of the first metal oxide nanoparticle by epitaxial growth.

6. The nanoparticle for radiation protection of claim 2, wherein oxygen vacancies of the surface of the first metal oxide nanoparticle are increased by the second metal oxide layer.

7. The nanoparticle for radiation protection of claim 2, wherein the second metal oxide layer is strained to expose {332} face.

8. The nanoparticle for radiation protection of claim 2, wherein the first metal oxide comprises ceria and the second metal oxide comprises manganese oxide.

9. The nanoparticle for radiation protection of claim 1, wherein the first metal oxide nanoparticle has oxygen vacancies.

10. The nanoparticle for radiation protection of claim 1, wherein the first metal oxide nanoparticle has a fluorite-structured nanocrystal.
